# EUROPEAN PATENT APPLICATION

(11) **EP 4 492 060 A1**
(43) Date of publication of application: **15.01.2025**
(21) Application number: 23763206.2
(22) Date of filing: 10.02.2023
(51) Int. Cl.: G01N 33/532, G01N 33/53, G01N 33/536

(54) **ANTI-PEG ANTIBODY BINDING MATERIAL AND METHOD FOR DETECTING ANTI-PEG ANTIBODY**

(30) Priority: 01.03.2022 JP 2022031256
(71) Applicant: Tokyo Institute of Technology, Tokyo 152-8550 (JP); DKS Co. Ltd., Kyoto-shi, Kyoto 600-8873 (JP)
(72) Inventor: SERIZAWA Takeshi, Tokyo 152-8550 (JP); SAWADA Toshiki, Tokyo 152-8550 (JP); MURASE Rina, kyoto-shi, Kyoto 600-8873 (JP); NISHIURA Masahito, Kyoto-shi, Kyoto 600-8873 (JP)
(74) Representative: Hoffmann Eitle
(86) International application number: PCT/JP2023/004480
(87) International publication number: WO 2023/166944

(57) **Abstract**

Provided is an anti-PEG-antibody-binding material capable of detecting a methoxy-terminated-PEG-selective anti-PEG antibody. The anti-PEG-antibody-binding material according to an embodiment contains a cellulose oligomer represented by formula (1) and is bindable to a methoxy-terminated-PEG-selective anti-PEG antibody: 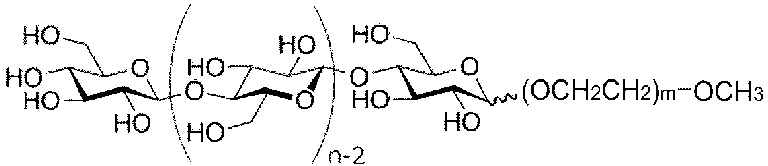 wherein n represents an average degree of polymerization, which is a number of 6 to 16, and m represents an integer of 1 to 16.

## Description

### Technical Field

The present invention relates to an anti-PEG-antibody-binding material and a method for detecting an anti-PEG antibody by using the material.

### Background Art

Modifying the surface of proteins, liposomes, micelles, or similar materials with polyethylene glycol (PEG) improves their blood retention and decreases their immunogenicity. Thus, many PEG-modified drugs are currently applied in clinical settings.

However, modification with PEG has been found to induce the secretion of antibodies against PEG (anti-PEG antibodies). If anti-PEG antibodies are present in blood, an administered PEG-modified drug is removed (ABC phenomenon) from the bloodstream, decreasing its blood retention or causing side effects. In order to assess the induction of anti-PEG antibodies and its impact on the therapeutic efficacy of PEG-modified drugs, anti-PEG antibodies present in blood must be detected with high sensitivity.

There are methoxy-terminated-PEG-selective anti-PEG antibodies, as well as backbone-selective anti-PEG antibodies that recognize the repeating structure of oxyethylene, which is the backbone of the PEG structure, and that are highly selective for the PEG chain of the backbone. Methoxy-terminated-PEG-selective anti-PEG antibodies are highly selective for methoxy-terminated PEG; i.e., these antibodies selectively bind to a PEG chain with a methoxy group at its end. Methoxy-terminated-PEG-selective anti-PEG antibodies are known to also be able to bind to a PEG chain with a more hydrophobic group, such as an ethoxy or butoxy group, at its end.

Techniques related to a biosensor containing a PEG chain include, for example, a biosensor containing a PEG-modified nanoparticle that is a metal (oxide) particle or semiconductor particle having a functional group or functional moiety via two or more PEG chains, as disclosed in PTL 1.

PTL 2 and PTL 3 disclose a biosensor that contains a hydrophilic particle composed of a water-insoluble particle, a primer layer containing a polysiloxane arranged on the surface of the particle, and a hydrophilic polymer layer arranged on the primer layer, in which the hydrophilic polymer layer is composed of a PEG chain having an antibody bound and a PEG chain having an inert group bound.

### Citation List

### Patent Literature

PTL 1: JP2005-180921A
PTL 2: JP2020-143964A
PTL 3: JP2021-012089A

### Non-patent Literature

NPL 1: T. Nohara et al., Enzymatic Synthesis of Oligo(ethylene glycol)-Bearing Cellulose Oligomers for in Situ Formation of Hydrogels with Crystalline Nanoribbon Network Structures, Langmuir, 2016, 32, 47, 12520-12526

### Summary of Invention

### Technical Problem

The present inventors conducted research with the aim of detecting anti-PEG antibodies present in blood with high sensitivity and found during the course of research that cellulose oligomers with a specific oligoethylene glycol group bind specifically to methoxy-terminated-PEG-selective anti-PEG antibodies.

Given the finding above, the embodiments of the present invention were made, and an object is to provide an anti-PEG-antibody-binding material capable of binding to a methoxy-terminated-PEG-selective anti-PEG antibody and a method for detecting an anti-PEG antibody using the material.

### Solution to Problem

The present invention includes the following embodiments.
[1] An anti-PEG-antibody-binding material capable of binding to a methoxy-terminated-PEG-selective anti-PEG antibody, comprising a cellulose oligomer represented by the following 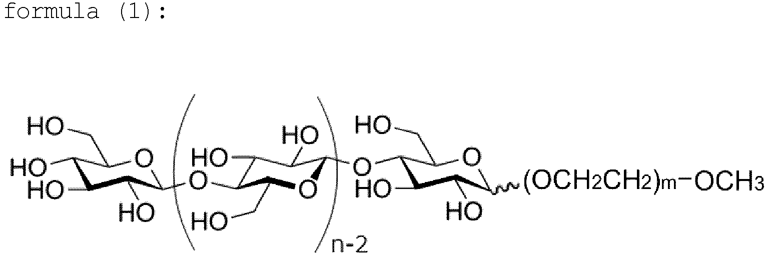 wherein n represents an average degree of polymerization, which is a number of 6 to 16, and m represents an integer of 1 to 16.
[2] A biosensor comprising the anti-PEG-antibody-binding material according to [1].
[3] An adsorbent comprising the anti-PEG-antibody-binding material according to [1].
[4] A method for detecting an anti-PEG antibody, comprising
   incubating a cellulose oligomer with a test sample suspected of containing the anti-PEG antibody under the conditions that the cellulose oligomer is bindable to a methoxy-terminated-PEG-selective anti-PEG antibody,
      the cellulose oligomer being represented by the following formula (1): 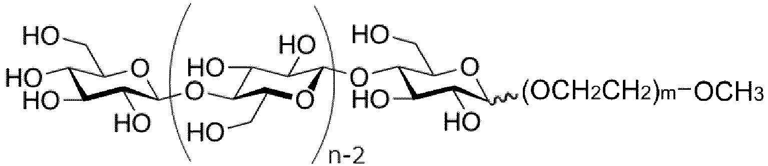
   wherein n represents an average degree of polymerization, which is a number of 6 to 16, and m represents an integer of 1 to 16;
   incubating the cellulose oligomer that has been incubated with the test sample with a reagent containing a secondary antibody under the conditions that the secondary antibody is bindable to the anti-PEG antibody; and
   detecting the anti-PEG antibody contained in the test sample with the secondary antibody as a probe by using the cellulose oligomer that has been incubated with the reagent.

### Advantageous Effects of Invention

The embodiments of the present invention provide an anti-PEG-antibody-binding material capable of binding to a methoxy-terminated-PEG-selective anti-PEG antibody. The anti-PEG-antibody-binding material has applications, for example, in biosensors for detecting a methoxy-terminated-PEG-selective anti-PEG antibody or in adsorbents for adsorbing a methoxy-terminated-PEG-selective anti-PEG antibody.

### Brief Description of Drawings

Fig. 1 is a conceptual diagram of a nanoribbon that is composed of a cellulose oligomer and that constitutes an anti-PEG-antibody-binding material according to an embodiment of the present invention.
Fig. 2 is an illustrative diagram showing a method for detecting an anti-PEG antibody according to an embodiment of the present invention.
Fig. 3 is a graph showing the results of evaluating the binding of a secondary antibody to each cellulose oligomer in Test Example 1 (absorbance at 490 nm).
Fig. 4 is a graph showing the results of evaluating the binding of a methoxy-terminated-PEG-selective anti-PEG antibody to each cellulose oligomer in Test Example 2 (absorbance at 490 nm) .
Fig. 5 is a graph showing the results of evaluating the binding of a backbone-selective anti-PEG antibody to each cellulose oligomer in Test Example 3 (absorbance at 490 nm).
Fig. 6 is a graph showing the results of BSA concentration dependence in blank measurement in Test Example 4 (absorbance at 490 nm).
Fig. 7 is a graph showing the results of BSA concentration dependence in blank measurement (with a cellulose oligomer) in Test Example 5 (absorbance at 490 nm).
Fig. 8 is a graph showing the results of evaluating the adsorption of BSA onto a cellulose oligomer in Test Example 6 (absorbance at 280 nm).
Fig. 9 is a chart showing the results of measuring the lower limit for detecting a methoxy-terminated-PEG-selective anti-PEG antibody in the presence of BSA in Test Example 7 (absorbance at 490 nm).
Fig. 10 is a chart showing an enlarged view of the low concentration range of the chart in Fig. 9.
Fig. 11 is a chart showing the results of detecting a methoxy-terminated-PEG-selective anti-PEG antibody in the presence of 10% FBS in Test Example 8 (absorbance at 490 nm).
Fig. 12 is a chart showing an enlarged view of the low concentration range of the chart in Fig. 11.
Fig. 13 is a chart showing the results of detecting a methoxy-terminated-PEG-selective anti-PEG antibody in the presence of 10% FBS in Test Example 9 (absorbance at 490 nm).
Fig. 14 is a chart showing an enlarged view of the low concentration range of the chart in Fig. 13.

### Description of Embodiments

The anti-PEG-antibody-binding material according to the present embodiment contains a cellulose oligomer represented by the following formula (1) ("OEGylated cellulose oligomer" below). The OEGylated cellulose oligomer has a structure in which a cellulose oligomer composed of glucose molecules linked via β-1,4-glucosidic bonds has as a substituent an oligoethylene glycol group having a methoxy group at the anomeric carbon of the reducing end.

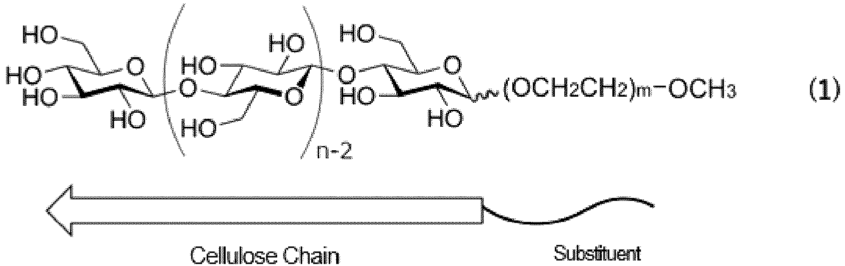

In formula (1), n represents a number of 6 to 16, and m represents an integer of 1 to 16. In formula (1), the wavy line indicates that the steric configuration of the anomeric position of the reducing end is α-form, β-form, or a mixture of α-form and β-form.

In formula (1), n indicates the average degree of polymerization (DP) of a cellulose oligomer, which is 6 or greater and 16 or lower; n may be 6.5 or greater or 7 or greater, and may be 14 or lower, 13 or lower, or 12 or lower. The average degree of polymerization of a cellulose oligomer indicates the weighted average value of the degree of polymerization according to the mass ratio of the cellulose oligomer. The degree of polymerization of each OEGylated cellulose oligomer is not particularly limited, and may be, for example, 4 or greater, 5 or greater, 6 or greater, and, for example, 20 or lower, 16 or lower, or 13 or lower.

In formula (1), m indicates the number of moles of ethylene oxide units added in the oligoethylene glycol group, and is 1 or greater and 16 or lower. In formula (1), m is preferably 3 or greater, and more preferably 4 or greater, and m is preferably 12 or lower, more preferably 10 or lower, and even more preferably 8 or lower. The OEGylated cellulose oligomer may be one with a single value of m ranging from 1 to 16, or may be a mixture with different values of m.

In an embodiment, the OEGylated cellulose oligomer may be represented by the following formula (2). In this case, the steric configuration of the anomeric position of the reducing end is β-form. In formula (2), m and n are the same as m and n in formula (1).

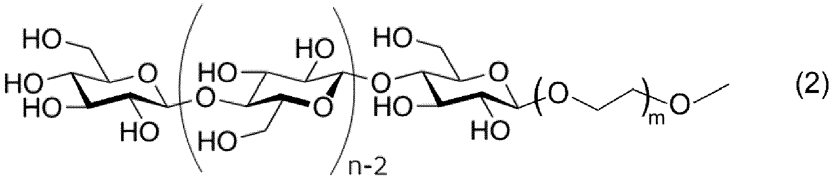

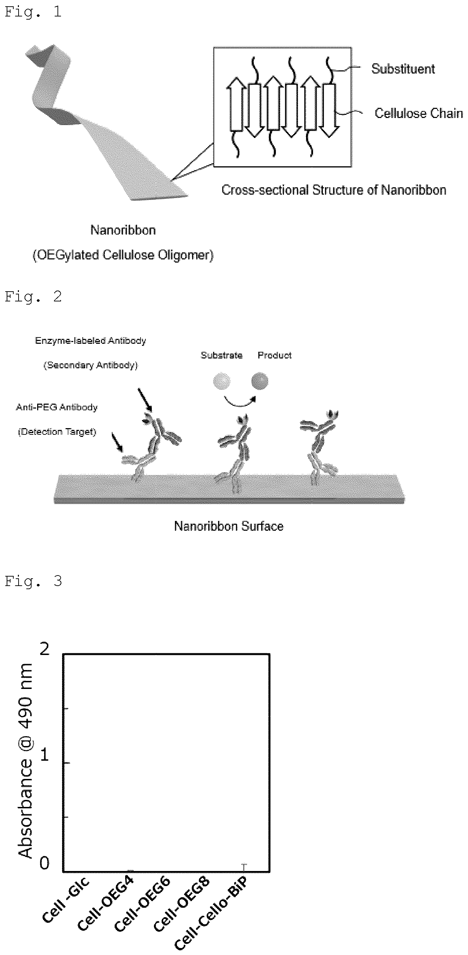

The OEGylated cellulose oligomer may be a cellulose oligomer assembly with a crystalline structure of cellulose II. Specifically, in an embodiment, the OEGylated cellulose oligomer may be a cellulose oligomer assembly with a crystalline structure of cellulose II, containing the compound represented by formula (1) as a constituent. The cellulose oligomer assembly may have a sheet structure (cellulose nanosheet). The concept of a sheet structure includes a ribbon-like structure (cellulose nanoribbon) as shown in Fig. 1.

Whereas natural-based cellulose chains have the crystalline structure of cellulose I aligned in parallel, artificially synthesized cellulose oligomers generally form a thermodynamically stable crystalline structure of cellulose II. During that formation, the substituents at the end of cellulose chains, (OC₂H₄)ₘ-OCH₃, do not affect the crystalline morphology. Cellulose oligomers with the substituent align in the film thickness direction of a cellulose nanosheet to form a lamellar crystal with the substituents exposed on the sheet surface.

For details, refer to the OEGylated cellulose oligomer depicted with an arrow from the reducing end to the non-reducing end in a cellulose chain and a substituent indicated by a wavy line, as noted under formula (1). OEGylated cellulose oligomers align in the film thickness direction so that their arrow directions alternate, resulting in the substituents exposed on the sheet surface alternately, as shown in the cross-sectional structure shown in Fig. 1.

The method for synthesizing the OEGylated cellulose oligomer is not particularly limited. For instance, the enzyme synthesis reaction using the reversed reaction of cellodextrin phosphorylase (CDP) disclosed in NPL 1 may be used. Specifically, a primer with the substituent via β-bond at the anomeric position of glucose or cellobiose or a primer with the substituent via α-bond at the anomeric position of cellobiose and α-glucose-1-phosphate (αG1P) are reacted with CDP to sequentially polymerize the αG1P to the primer, thereby synthesizing the OEGylated cellulose oligomer represented by formula (1).

The OEGylated cellulose oligomer represented by formula (1) specifically binds to a methoxy-terminated-PEG-selective anti-PEG antibody and does not bind to other proteins, such as a secondary antibody used as a probe, bovine serum albumin (BSA), and lysozyme. Thus, the cellulose oligomer assembly has a bio-inert surface not nonspecifically adsorbing proteins and specifically binds to a methoxy-terminated-PEG-selective anti-PEG antibody. The OEGylated cellulose oligomer also does not bind to backbone-selective anti-PEG antibodies. Because of its capability of selectively binding to a methoxy-terminated-PEG-selective anti-PEG antibody, the OEGylated cellulose oligomer can be used as an anti-PEG-antibody-binding material capable of selectively detecting or adsorbing a methoxy-terminated-PEG-selective anti-PEG antibody.

The methoxy-terminated-PEG-selective anti-PEG antibody, to which the OEGylated cellulose oligomer is bindable, is more specifically a monoclonal antibody that specifically binds to PEG (polyethylene glycol) with a methoxy group at an end, and is also referred to as a "methoxy-terminated-PEG-selective monoclonal anti-PEG antibody." The methoxy-terminated-PEG-selective anti-PEG antibody may also be bindable to a PEG chain with a more hydrophobic group such as an ethoxy or butoxy group at its end. The class of the methoxy-terminated-PEG-selective anti-PEG antibody is not particularly limited, and may be IgG or IgM.

Because of the OEGylated cellulose oligomer contained, the anti-PEG-antibody-binding material according to the present embodiment can recognize and bind to a methoxy-terminated-PEG-selective anti-PEG antibody. Thus, the anti-PEG-antibody-binding material has applications, for example, in biosensors for detecting a methoxy-terminated-PEG-selective anti-PEG antibody or in adsorbents for adsorbing a methoxy-terminated-PEG-selective anti-PEG antibody.

The anti-PEG-antibody-binding material may be composed of only an OEGylated cellulose oligomer (e.g., the cellulose oligomer assembly described above), or may additionally contain other components, such as proteins, peptides, nucleic acids, lipids, sugars, cells, amino acids, metal ions, and organic solvents, to the extent that the effects are not impaired.

The OEGylated cellulose oligomer, specifically its assembly (cellulose oligomer assembly), is a substance that does not dissolve in water. When synthesized by the enzymatic synthesize reaction described above, the OEGylated cellulose oligomer is obtained in the form of an aqueous dispersion. The anti-PEG-antibody-binding material according to an embodiment may be a dry powder obtained by removing water from the aqueous dispersion. The dry powder may be used as is or mixed with other components such as proteins to prepare a powdered anti-PEG-antibody-binding material. The powdered anti-PEG-antibody-binding material can easily be dispersed in water by adding water to prepare an aqueous dispersion when used.

The anti-PEG-antibody-binding material according to an embodiment may also be an aqueous dispersion obtained by dispersing an OEGylated cellulose oligomer in water. In the preparation of an aqueous dispersion of an OEGylated cellulose oligomer, a buffering agent may be added to water to form a buffer solution. Specifically, the aqueous dispersion of an OEGylated cellulose oligomer may also be a dispersion of an OEGylated cellulose oligomer in the buffer solution.

Examples of buffering agents include, but are not particularly limited to, those forming the following: phosphate buffered saline (PBS), 2-morpholinoethanesulfonic acid (MES) buffer solutions, trishydroxymethylaminomethane (Tris) buffer solutions, 3-morpholinopropanesulfonic acid (MOPS) buffer solutions, and 4-(2-hydroxyethyl)-1-piperazineethanesulfonic acid (HEPES) buffer solutions.

For an aqueous dispersion of an OEGylated cellulose oligomer, the content (concentration) of the OEGylated cellulose oligomer is not particularly limited, and may be, for example, 0.01 to 5% (w/v), or 0.03 to 0.3% (w/v).

In the present specification, "% (w/v)" represents a concentration by mass/volume percent, which is the mass (g) of a target substance per 100 mL of volume.

The anti-PEG-antibody-binding material according to an embodiment may be an OEGylated cellulose oligomer (e.g., the cellulose oligomer assembly described above) immobilized on the surface of a substrate, such as plastic or glass.

In an embodiment, a biosensor containing the anti-PEG-antibody-binding material is used to perform a bioassay for detecting and quantifying a methoxy-terminated-PEG-selective anti-PEG antibody. The biosensor refers to each element or an assembly or combination of the elements used to perform the bioassay.

Thus, the biosensor may be composed of only a powder of an OEGylated cellulose oligomer or of only an aqueous dispersion of an OEGylated cellulose oligomer. Alternatively, the biosensor may be a kit that combines a powder or aqueous dispersion of an OEGylated cellulose oligomer with a secondary antibody (probe) (e.g., an anti-PEG antibody detection kit). Additionally, for instance, if the secondary antibody is an enzyme-labeled antibody, the biosensor may be an anti-PEG antibody detection kit that also contains a substrate for the enzyme in combination.

As demonstrated in the Examples described below, the detection sensitivity of the OEGylated cellulose oligomer for a methoxy-terminated-PEG-selective anti-PEG antibody can be improved by adding albumin, such as bovine serum albumin (BSA), when the OEGylated cellulose oligomer is incubated with the test sample.

Thus, the biosensor may contain albumin, such as BSA; it is preferred that albumin be added when an OEGylated cellulose oligomer and a test sample are incubated. In that case, albumin may be contained in the anti-PEG-antibody-binding material or may be included in the anti-PEG antibody detection kit, separately from the anti-PEG-antibody-binding material. If albumin is contained in the anti-PEG-antibody-binding material, the OEGylated cellulose oligomer and albumin may be mixed in powder form, or albumin may be dissolved in an aqueous dispersion of an OEGylated cellulose oligomer. There is no particular limitation to the ratio of the OEGylated cellulose oligomer to albumin. For example, the ratio by mass of the OEGylated cellulose oligomer to albumin may be 0.003/1 to 0.3/1, or 0.01/1 to 0.1/1. If the test sample contains albumin, such as blood, additional albumin may be or may not be added.

The method for detecting an anti-PEG antibody according to an embodiment comprises the following steps of:
(1) incubating an OEGylated cellulose oligomer with a test sample under the conditions that the OEGylated cellulose oligomer is bindable to a methoxy-terminated-PEG-selective anti-PEG antibody,
(2) incubating the OEGylated cellulose oligomer that has been incubated with the test sample with a reagent containing a secondary antibody under the conditions that the secondary antibody is bindable to the anti-PEG antibody, and
(3) detecting the methoxy-terminated-PEG-selective anti-PEG antibody contained in the test sample with the secondary antibody as a probe by using the OEGylated cellulose oligomer that has been incubated with the reagent.

In step (1), the test sample is added to a container together with an aqueous dispersion of the OEGylated cellulose oligomer as the anti-PEG-antibody-binding material, and then mixed and dispersed, followed by incubation.

The test sample is a substance to be tested, suspected of containing a methoxy-terminated-PEG-selective anti-PEG antibody. For example, if anti-PEG antibodies in blood are to be detected to assess the induction of anti-PEG antibodies and its impact on the therapeutic efficacy of PEG-modified drugs, blood or a diluent of blood may be used as a test sample.

In step (1), as described above, albumin, such as BSA, may be added to enhance the detection sensitivity for a methoxy-terminated-PEG-selective anti-PEG antibody. In that case, the concentration of albumin in the system (the concentration of albumin excluding the albumin originally contained in the test sample) is preferably, but not particularly limited to, for example, 0.05 to 100 (w/v), and more preferably 0.5 to 5% (w/v).

The incubation conditions in step (1) are not particularly limited as long as the OEGylated cellulose oligomer is bindable to a methoxy-terminated-PEG-selective anti-PEG antibody. For instance, the incubation conditions may be 4°C to 60°C, and 1 minute to 2 hours. The concentration of the OEGylated cellulose oligomer in the system is not particularly limited and may be, for example, 0.01 to 1% (w/v), or 0.01 to 0.1% (w/v).

In step (1), the methoxy-terminated-PEG-selective anti-PEG antibody, which is the detection target, is conjugated with the OEGylated cellulose oligomer in this manner (see Fig. 2). Subsequently, centrifugation and washing are performed to separate the OEGylated cellulose oligomer conjugated with the methoxy-terminated-PEG-selective anti-PEG antibody from the test sample.

In step (2), a reagent containing a secondary antibody is added to the OEGylated cellulose oligomer conjugated with the methoxy-terminated-PEG-selective anti-PEG antibody, mixed and dispersed, and then incubated.

Examples of reagents containing a secondary antibody include aqueous solutions of a secondary antibody. The secondary antibody can be any antibody that is specifically bindable to a methoxy-terminated-PEG-selective anti-PEG antibody so as to function as a probe. Examples include antibodies labeled with an enzyme, a radioactive isotope, biotin, a fluorescent dye, etc.

The incubation conditions in step (2) are not particularly limited as long as the methoxy-terminated-PEG-selective anti-PEG antibody is bindable to the secondary antibody. For instance, the incubation conditions may be 4°C to 60°C, and 1 minute to 2 hours. The concentration of the secondary antibody in the system is not particularly limited and may be, for example, 100 to 1000 ng/mL.

In step (2), a secondary antibody (probe) is bonded with the anti-PEG antibody conjugated with the OEGylated cellulose oligomer in this manner (see Fig. 2). Subsequently, centrifugation and washing are performed to separate the OEGylated cellulose oligomer conjugated with the secondary antibody from the reagent containing the secondary antibody.

In step (3), the OEGylated cellulose oligomer conjugated with the secondary antibody is used to detect and quantify a methoxy-terminated-PEG-selective anti-PEG antibody contained in the test sample by using the secondary antibody as a probe.

For example, when an enzyme-labeled antibody is used as a secondary antibody, a substrate for the enzyme is added to the OEGylated cellulose oligomer conjugated with the secondary antibody to perform an enzyme reaction, as shown in Fig. 2. The pigment of the generated product is detected and quantified by absorption (color), fluorescence, chemiluminescence, etc.

In another embodiment, an adsorbent containing the anti-PEG-antibody-binding material can be used to adsorb the methoxy-terminated-PEG-selective anti-PEG antibody to thereby remove or purify the anti-PEG antibody.

The adsorbent may be formed of a powder of the OEGylated cellulose oligomer or an aqueous dispersion of the OEGylated cellulose oligomer, and may also contain other optional components.

In the use of the adsorbent in removing a methoxy-terminated-PEG-selective anti-PEG antibody, for example, the adsorbent is added to a liquid containing the anti-PEG antibody to bring the anti-PEG antibody into contact with the OEGylated cellulose oligomer and bind them together. Thereafter, the adsorbent that has adsorbed the anti-PEG antibody is removed from the liquid using a technique such as centrifugation to remove the anti-PEG antibody from the liquid.

In the use of the adsorbent in purifying a methoxy-terminated-PEG-selective anti-PEG antibody, for example, the adsorbent is added to a liquid containing impurities together with the anti-PEG antibody to bring the anti-PEG antibody into contact with the OEGylated cellulose oligomer and bind them together. Thereafter, the OEGylated cellulose oligomer that has adsorbed the anti-PEG antibody is removed and collected from the liquid using a technique such as centrifugation. The collected OEGylated cellulose oligomer is then subjected to, for example, acid treatment, which releases the bond between the anti-PEG antibody and the OEGylated cellulose oligomer. This enables the separation and collection of the anti-PEG antibody from the OEGylated cellulose oligomer and thus provides a high-purity methoxy-terminated-PEG-selective anti-PEG antibody.

### Examples

The following describes in more detail the present invention with reference to Examples. However, the invention is not limited to the Examples.

### Preparation of Non-modified Cellulose Oligomer (Cell-Glc)

Following the method reported by T. Serizawa et al., Polym. J., 2016, 48, 539-544, an unmodified cellulose oligomer (Cell-Glc) represented by the following formula with an average degree of polymerization of 10 (n=10) was synthesized using glucose as a primer.

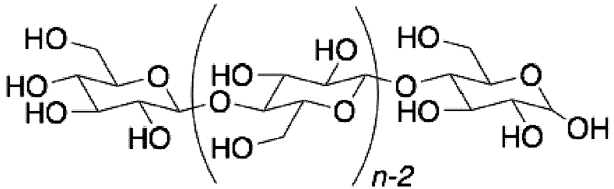

### Preparation of OEGylated Cellulose Oligomer (Cell-OEGm)

Following the method reported in NPL 1 (T. Nohara et al., Langmuir, 2016, 32, 47, 12520-12526), OEGylated cellulose oligomers presented by formula (2) were synthesized using OEGylated glucose as a primer. The OEGylated cellulose oligomers synthesized were the following: a cellulose oligomer (n=10, m=4 in formula (2)) referred to as "Cell-OEG4," a cellulose oligomer (n=10, m=6 in formula (2)) referred to as "Cell-OEG6," and cellulose oligomer (n=11, m=8 in formula (2)) referred to as "Cell-OEG8."

The average degree of polymerization was calculated based on the integral values of protons at the anomeric position other than the reducing end (near δ4.2 ppm) and at the anomeric position of the reducing end (near δ4.3 ppm) in the cellulose moiety of the OEGylated cellulose oligomers by using a proton nuclear magnetic resonance (NMR) instrument (AVANCE III HD500 (Bruker Biospin, magnetic field intensity: 500 MHz, cumulated number: 16)).

### Preparation of Cellulose Oligomer (Cell-Cello-BiP)

Following the method reported by K. Sugiura et al., Polym. J., 2021, 53.1133, a cellulose oligomer represented by the following formula ("Cell-Cello-BiP," p=5, q=5) was synthesized using an oligoethylene glycol having cellobiose at each end as a primer.

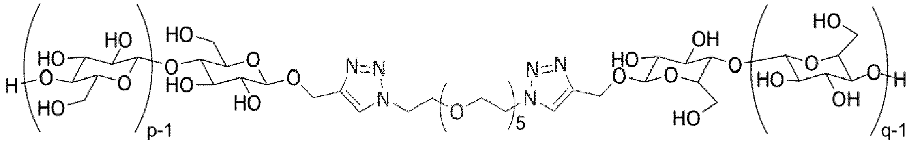

### Reagents

The reagents BSA and OPD used in the Test Examples are the following:
BSA: Bovine serum albumin, Fujifilm Wako Pure Chemical Corporation, free from proteases.
OPD: o-phenylenediamine dihydrochloride, manufactured by Nacalai Tesque, Inc., for water quality analysis

### Test Example 1: Evaluation of Binding of Secondary Antibody

The adsorption of a secondary antibody to be used in evaluating the binding of anti-PEG antibodies was evaluated. For the secondary antibody, an anti-rabbit IgG, HRP-labeled antibody (manufactured by GeneTex) was used. The antibody was diluted 1000-fold with a 3.3% (w/v) BSA/PBS solution to prepare a 410 ng/mL solution of the secondary antibody. The 3.3% (w/v) BSA/PBS solution was prepared by dissolving 330 mg of BSA in 10 mL of PBS (pH 7.4, the same applies below).

A cellulose oligomer was dispersed in PBS at a concentration of 5 mg/mL to prepare a cellulose dispersion. The cellulose oligomers used in preparing various cellulose dispersions were the following: an unmodified cellulose oligomer (Cell-Glc), OEGylated cellulose oligomers (Cell-OEG4, Cell-OEG6, and Cell-OEG8), and a cellulose oligomer (Cell-Cello-BiP).

### 1. Adsorption of Secondary Antibody

10 µL of a cellulose dispersion was added to a 0.2 mL PCR tube, and 100 µL of the secondary antibody solution was further added and dispersed, followed by incubation at 25°C for 1 hour. Subsequently, centrifugation was performed (15000 rpm, 25°C, 5 minutes) to remove the supernatant, after which, 100 µL of PBS was added for redispersion. This cycle of centrifugation and redispersion was performed three times for washing.

### 2. Enzyme Reaction

After removal of the supernatant by centrifugation, 100 µL of a substrate solution was added to the PCR tube. The substrate solution used was a citrate-phosphate buffer (pH: 4.0) containing 2 mg/mL OPD and 0.11 v/v% hydrogen peroxide. The substrate solution was added and dispersed, followed by incubation at 25°C for 30 minutes. Subsequently, 100 µL of 3N sulfuric acid was added, and the mixture was centrifuged (15000 rpm, 25°C, 10 minutes). Thereafter, 100 µL of the supernatant was added to a 96-well plate, and the absorbance at 490 nm was measured with a plate reader (Synergy H1, manufactured by BioTek).

The results are as shown in Fig. 3. The HRP-labeled antibody (secondary antibody) was adsorbed to none of the unmodified cellulose oligomer (Cell-Glc), OEGylated cellulose oligomers (Cell-OEG4, Cell-OEG6, Cell-OEG8), and cellulose oligomer (Cell-Cello-BiP).

### Test Example 2: Evaluation of Binding of Methoxy-terminated-PEG-selective Anti-PEG Antibody

The methoxy-terminated-PEG-selective anti-PEG antibody (methoxy-mAb) used as a primary antibody was PEG-B-47 (manufactured by Abcam Limited). PEG-B-47 is a methoxy-terminated-PEG-selective monoclonal anti-PEG antibody (animal species: rabbit, class: IgG, and immunogen: PEG-KLH; Merry R. Sherman et al., Molecular Immunology, 2008, 57, 236-246). PEG-B-47 was diluted 68500-fold with a 3.3% (w/v) BSA/PBS solution to prepare a primary antibody solution containing 11 ng/mL PEG-B-47.

The cellulose dispersion, secondary antibody solution, and substrate solution were the same as those used in Test Example 1.

### 1. Antigen-antibody Reaction of Primary Antibody

10 µL of a cellulose dispersion (5 mg/mL, PBS) was added to a 0.2 mL PCR tube, and 100 µL of a primary antibody solution was further added. After being dispersed, the mixture was incubated at 25°C for 1 hour to allow an antigen-antibody reaction to proceed. The antigen-antibody reaction system had a cellulose oligomer concentration of 0.45 mg/mL, a primary antibody concentration of 10 ng/mL, and a BSA concentration of 3% (w/v).

After incubation, centrifugation was performed (15000 rpm, 25°C, 5 minutes) to remove the supernatant, followed by redispersion in 100 µL of PBS. This cycle of centrifugation and redispersion was performed three times for washing.

### 2. Antigen-antibody Reaction of Secondary Antibody

Subsequently, centrifugation (15000 rpm, 25°C, 5 minutes) was performed to remove the supernatant. 100 µL of a secondary antibody solution (secondary antibody content: 410 ng/mL of a 3.3% BSA/PBS solution) was added to the PCR tube and dispersed, followed by incubation at 25°C for 1 hour. Thereafter, centrifugation was performed (15000 rpm, 25°C, 5 minutes) to remove the supernatant, followed by redispersion in 100 µL of PBS. This cycle of centrifugation and redispersion was performed three times for washing.

### 3. Enzyme Reaction

Subsequently, centrifugation (15000 rpm, 25°C, 5 minutes) was performed to remove the supernatant, and then 100 µL of a substrate solution (OPD: 2 mg/mL, H₂O₂: 0.11 v/v%, citrate-phosphate buffer with a pH of 4.0) was added to the PCR tube and dispersed, followed by incubation at 25°C for 30 minutes. Thereafter, 100 µL of 3N sulfuric acid was added, followed by centrifugation (15000 rpm, 25°C, 10 minutes). The supernatant was taken and diluted two-fold with ultrapure water, and then 100 µL of the diluted supernatant was added to a 96-well plate. The absorbance at 490 nm was measured with a plate reader (Synergy H1, manufactured by BioTek).

From the measured absorbance of the individual cellulose oligomer, the absorbance measured in the same manner without a cellulose oligomer in an experiment was subtracted to determine the absorbance for evaluation. The experiment was performed three times for each cellulose oligomer, and the average absorbance was calculated.

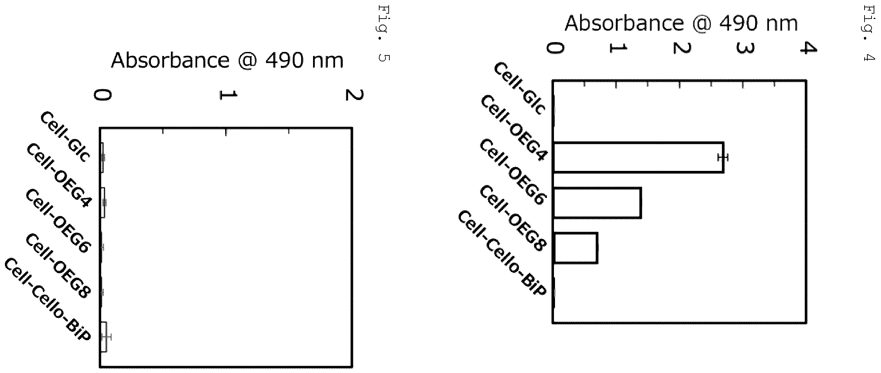

The results are as shown in Fig. 4. It is clear that practically no methoxy-terminated-PEG-selective anti-PEG antibody was bound to the unmodified cellulose oligomer (Cell-Glc) and to the cellulose oligomer (Cell-Cello-BiP).

In contrast, the results indicate that the methoxy-terminated-PEG-selective anti-PEG antibody was bound to the OEGylated cellulose oligomers (Cell-OEG4, Cell-OEG6, Cell-OEG8). The fact that the HRP-labeled antibody (secondary antibody) was not adsorbed to these cellulose oligomers in Test Example 1 supports specific binding of the methoxy-terminated-PEG-selective anti-PEG antibody to the OEGylated cellulose oligomers.

The amount of the anti-PEG antibody bound to Cell-OEG4, among the OEGylated cellulose oligomers, was highest. The binding amount of the anti-PEG antibody decreased with an increase in the number of moles of the ethylene oxide unit added to the oligoethylene glycol group.

### Test Example 3: Evaluation of Binding of Backbone-selective Anti-PEG Antibody

The backbone-selective anti-PEG antibody (backbone-mAb) used as a primary antibody was clone 6.3 (manufactured by Merck, animal species: Mouse, class: IgG1). Clone 6.3 is known to bind to PEGs of 750 Da or greater (Samuel K. Lai, Commun. Chem., 2020, 3, 124). Clone 6.3 was diluted 145500-fold with a 3.3% (w/v) BSA/PBS solution to prepare a primary antibody solution containing 11 ng/mL clone 6.3.

A secondary antibody for use, which was an anti-mouse IgG1, HRP-labeled antibody (manufactured by Abcam Limited), was diluted 2273-fold with a 3.3% (w/v) BSA/PBS solution to prepare a secondary antibody solution containing 440 ng/mL of the secondary antibody. The cellulose dispersion and substrate solution used were the same as those in Test Example 1.

### 1. Antigen-antibody Reaction of Primary Antibody

10 µL of a cellulose dispersion was added to a 0.2 mL PCR tube, and then 100 ul of a primary antibody solution was further added. After being dispersed, the mixture was incubated at 25°C for 1 hour to allow an antigen-antibody reaction to proceed. The antigen-antibody reaction system had a cellulose oligomer concentration of 0.45 mg/ml, a primary antibody concentration of 10 ng/ml, and a BSA concentration of 3% (w/v). After incubation, centrifugation was performed (15000 rpm, 25°C, 5 minutes), and the supernatant was removed, followed by redispersion in 100 µL of PBS. This cycle of centrifugation and redispersion was performed three times for washing.

### 2. Antigen-antibody Reaction of Secondary Antibody

Subsequently, centrifugation (15000 rpm, 25°C, 5 minutes) was performed to remove the supernatant. Thereafter, 100 µL of the secondary antibody solution was added to the PCR tube and dispersed, followed by incubation at 25°C for 1 hour. Thereafter, centrifugation was performed (15000 rpm, 25°C, 5 minutes) to remove the supernatant, followed by redispersion in 100 µL of PBS. This cycle of centrifugation and redispersion was performed three times for washing.

### 3. Enzyme Reaction

After removal of the supernatant by centrifugation (15000 rpm, 25°C, 5 minutes), 100 µL of a substrate solution (OPD: 2 mg/mL, H₂O₂: 0.11 v/v%, a citrate-phosphate buffer with a pH of 4.0) was added to the PCR tube and dispersed, followed by incubation at 25°C for 30 minutes. Subsequently, 100 µL of 3N sulfuric acid was added, and the mixture was centrifuged (15000 rpm, 25°C, 10 minutes). Thereafter, 100 µL of the supernatant was added to a 96-well plate, and then the absorbance at 490 nm was measured with a plate reader (Synergy H1, manufactured by BioTek).

From the measured absorbance of the individual cellulose oligomer, the absorbance measured in the same manner without a cellulose oligomer in an experiment was subtracted to determine the absorbance for evaluation. The experiment was conducted in three sets, three times for each, and the average absorbance was calculated from the average values of the three sets.

The results are as shown in Fig. 5. The absorbance determined by subtracting the control absorbance was zero, indicating that the backbone-selective-anti-PEG antibody was bound to none of the OEGylated cellulose oligomers (Cell-OEG4, Cell-OEG6, and Cell-OEG8). Thus, the cellulose oligomer according to the present embodiment is thought to be able to selectively detect a methoxy-terminated-PEG-selective anti-PEG antibody.

### Test Example 4: BSA Concentration Dependence in Blank Measurement

A secondary antibody that is non-specifically adsorbed to a container decreases detection sensitivity. In order to decrease the non-specific adsorption of a secondary antibody to a container, BSA, which is protein inert to antigen-antibody reaction, was allowed to be present in a reaction system to study the impact.

### 1. Adsorption of BSA

10 µl of PBS was added to a 0.2 ml PCR tube, and 100 µL of a BSA solution was further added and dispersed. The BSA solutions used were the following: a 0.11% (w/v) BSA/PBS solution prepared by dissolving 1.1 mg of BSA in 1 mL of PBS, a 0.33% (w/v) BSA/PBS solution prepared by dissolving 3.3 mg of BSA in 1 mL of PBS, a 1.1% (w/v) BSA/PBS solution prepared by dissolving 11 mg of BSA in 1 mL of PBS, a 3.3% (w/v) BSA/PBS solution prepared by dissolving 33 mg of BSA in 1 mL of PBS, and a 0% (w/v) PBS solution with no BSA added.

After a BSA solution was added and dispersed, the mixture was incubated at 25°C for 1 hour. Thereafter, the content was replaced three times with 100 µL of PBS to wash the PCR tube.

### 2. Adsorption of Secondary Antibody

Subsequently, 100 µL of a secondary antibody solution (secondary antibody content: 410 ng/mL in a 3.3% BSA/PBS solution) was added to the washed PCR tube, and the mixture was incubated at 25°C for 1 hour. The secondary antibody solution used was the same as in Test Example 1.

After incubation, the content was replaced three times with 100 µL of PBS to wash the PCR tube.

### 3. Enzyme Reaction

Subsequently, after removal of PBS, 100 µL of a substrate solution (OPD: 2 mg/mL, H₂O₂: 0.11 v/v%, a citrate-phosphate buffer with a pH of 4.0) was added to the PCR tube, and the mixture was incubated at 25°C for 30 minutes. The substrate solution used was the same as in Test Example 1. Thereafter, 100 µL of 3N sulfuric acid was added.

Subsequently, 100 µL of the solution that underwent enzyme reaction was dispensed into a 96-well plate, and the absorbance at 490 nm was measured with a plate reader (Synergy H1, manufactured by BioTek). The experiment was performed three times, and the average absorbance of the three experiments was calculated.

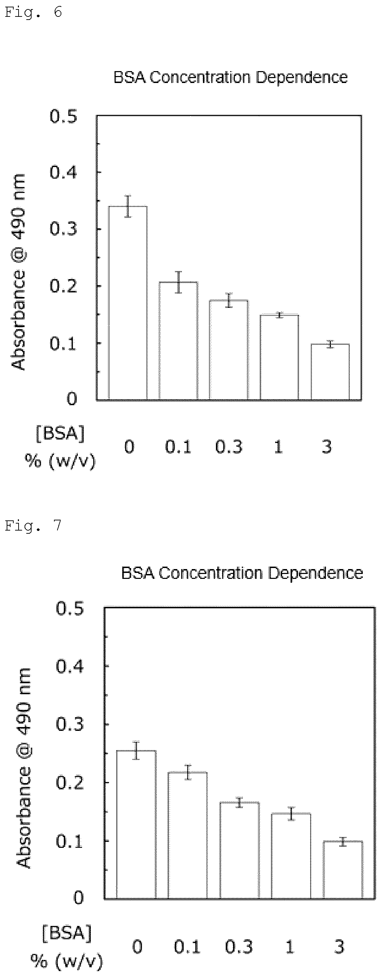

The results are as shown in Fig. 6. With an increase in the concentration of BSA, the absorbance decreased, and the background decreased. This is probably because the adsorption of BSA onto the container (tube) limited non-specific adsorption of the secondary antibody.

### Test Example 5: BSA Concentration Dependence in Blank Measurement (with Cellulose Oligomer)

A study was conducted to determine the effect of the BSA concentration on non-specific adsorption of the secondary antibody when cellulose oligomers were added.

### 1. Adsorption of BSA

10 µL of a cellulose dispersion was added to a 0.2 mL PCR tube, and then 100 µL of a BSA solution was further added and dispersed. The cellulose dispersion used was prepared by dispersing an OEGylated cellulose oligomer (Cell-OEG 4) in PBS at a concentration of 5 mg/mL. The BSA solutions used were the same five solutions as those in Test Example 4.

After the BSA solution was added and dispersed, the mixture was incubated at 25°C for 1 hour, followed by centrifugation (15000 rpm, 25°C, 5 minutes) to remove the supernatant. Thereafter, redispersion in 100 µL of PBS was performed. This cycle of centrifugation and redispersion was performed three times for washing.

### 2. Adsorption of Secondary Antibody

Subsequently, after removal of the supernatant by centrifugation (15000 rpm, 25°C, 5 minutes), 100 µL of a secondary antibody solution (secondary antibody content: 410 ng/mL in a 3.3% BSA/PBS solution) was added to the PCR tube and dispersed, followed by incubation at 25°C for 1 hour. The secondary antibody solution used was the same as in Test Example 4.

After incubation, centrifugation was performed (15000 rpm, 25°C, 5 minutes) to remove the supernatant, followed by redispersion in 100 µL of PBS. This cycle of centrifugation and redispersion was performed three times for washing.

### 3. Enzyme Reaction

Subsequently, centrifugation (15000 rpm, 25°C, 5 minutes) was performed to remove the supernatant, and 100 µL of a substrate solution (OPD: 2 mg/mL, H₂O₂: 0.11 v/v%, a citrate-phosphate buffer with a pH of 4.0) was added to the PCR tube and dispersed, followed by incubation at 25°C for 30 minutes. The substrate solution used was the same as in Test Example 4. Subsequently, 100 µL of 3N sulfuric acid was added, followed by centrifugation (15000 rpm, 25°C, 10 minutes).

Subsequently, 100 µL of the supernatant was dispensed into a 96-well plate, and the absorbance at 490 nm was measured with a plate reader (Synergy H1, manufactured by BioTek). The experiment was performed three times, and the average absorbance of the three experiments was calculated.

The results are as shown in Fig. 7. With an increase in the concentration of BSA, the absorbance decreased, and the background decreased. The results indicate that adding a high concentration of BSA and performing incubation before treatment with a secondary antibody can limit non-specific adsorption of the secondary antibody to be added to the container and thus improve detection sensitivity (S/N ratio).

### Test Example 6: Evaluation of Adsorption of BSA onto Cellulose Oligomer

48 µL of a cellulose dispersion was added to a 0.2 mL of PCR tube, and 72 µL of a BSA solution was further added and dispersed. The cellulose dispersion used was prepared by dispersing an OEGylated cellulose oligomer (Cell-OEG4) in PBS at a concentration of 5 mg/mL. The BSA solution used was a 5% (w/v) BSA/PBS solution prepared by dissolving 50 mg of BSA in 1 mL of PBS. In the mixture of both, the concentration of the cellulose oligomer was 2 mg/mL, and the concentration of BSA was 3% (w/v).

After mixing, the mixture was incubated at 25°C for 1 hour, followed by centrifugation (15000 rpm, 25°C, 5 minutes). Thereafter, 25 µL of the supernatant was taken and diluted 20-fold with ultrapure water. 50 µL of the diluted supernatant was dispensed into a microcell.

UV-vis (ultraviolet-visible) spectroscopy was then performed with a wavelength of 200 to 800 nm to determine the absorbance at 280 nm. The measurement was performed three times, and the average value was calculated.

For a blank, 48 µL of PBS free of a cellulose oligomer (Cell-OEG4), instead of the cellulose dispersion, was added, and the average absorbance was determined in the same manner as above.

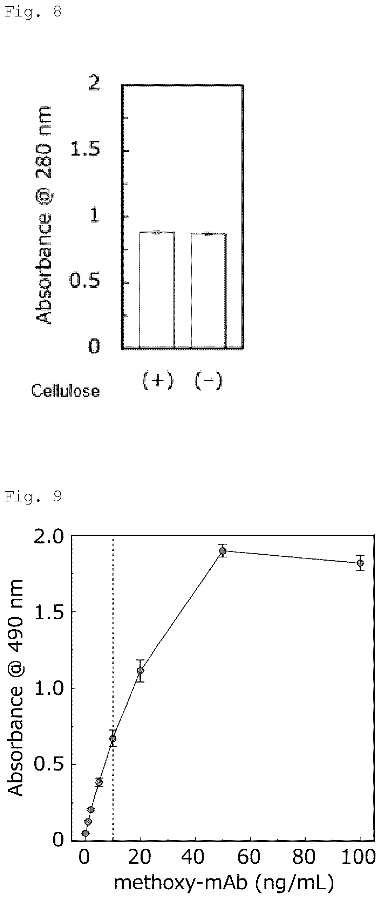

The results are as shown in Fig. 8. In Fig. 8, "Cellulose (+)" denotes the example using a cellulose dispersion, whereas "Cellulose (-)" denotes the example using PBS free of a cellulose oligomer (Cell-OEG4) as the blank.

As shown in Fig. 8, the absorbance was almost the same regardless of whether the cellulose oligomer (Cell-OEG4) was added or not. This indicates that BSA is hardly adsorbed to the cellulose oligomer (Cell-OEG4).

### Test Example 7: Detection of Methoxy-terminated-PEG-selective anti-PEG antibody in the Presence of BSA

The cellulose dispersion for use was prepared by dispersing an OEGylated cellulose oligomer (Cell-OEG4) in PBS at a concentration of 5 mg/mL.

The primary antibody solutions for use were prepared by diluting PEG-B-47 with a 3.3% (w/v) BSA/PBS solution so that the primary antibody had the following concentrations: 1.1 ng/mL, 2.2 ng/mL, 5.5 ng/mL, 11 ng/mL, 22 ng/mL, 55 ng/mL, and 110 ng/mL (diluted 6850-fold).

The secondary antibody solution and substrate solution were the same as those used in Test Example 1.

### 1. Antigen-antibody Reaction of Primary Antibody

10 µL of a cellulose dispersion (Cell-OEG4, 5 mg/mL, PBS) was added to a 0.2 mL PCR tube, and 100 µL of a primary antibody solution was added and dispersed. Thereafter, the mixture was incubated at 25°C for 1 hour to allow an antigen-antibody reaction to proceed. The antigen-antibody reaction system had a cellulose oligomer concentration of 0.45 mg/mL, a primary antibody concentration of 1 to 100 ng/mL, and a BSA concentration of 3% (w/v). For a blank control, 100 µL of a 3.3% (w/v) BSA/PBS solution containing no primary antibody was added and incubated for 1 hour.

After incubation, centrifugation was performed (15000 rpm, 25°C, 5 minutes) to remove the supernatant, followed by redispersion in 100 µL of PBS. This cycle of centrifugation and redispersion was performed three times for washing.

### 2. Antigen-antibody Reaction of Secondary Antibody

Subsequently, centrifugation was performed (15000 rpm, 25°C, 5 minutes) to remove the supernatant, and 100 µL of a secondary antibody solution (secondary antibody content: 410 ng/ml in a 3.3% BSA/PBS solution) was added to the PCR tube and dispersed, followed by incubation at 25°C for 1 hour. Thereafter, centrifugation was performed (15000 rpm, 25°C, 5 minutes) to remove the supernatant, followed by redispersion in 100 µL of PBS. This cycle of centrifugation and redispersion was performed three times for washing.

### 3. Enzyme Reaction

Subsequently, after centrifugation was performed (15000 rpm, 25°C, 5 minutes) to remove the supernatant, 100 µL of a substrate solution (OPD: 2 mg/mL, H₂O₂: 0.11 v/v%, a citrate-phosphate buffer with a pH of 4.0) was added to the PCR tube and dispersed, followed by incubation at 25°C for 30 minutes. Thereafter, 100 µL of 3N sulfuric acid was added, and the mixture was centrifuged (15000 rpm, 25°C, 10 minutes). The supernatant was taken and diluted 5-fold with ultrapure water. 100 µL of the diluted supernatant was then dispensed into a 96-well plate, and the absorbance at 490 nm was measured with a plate reader (Synergy H1, manufactured by BioTek).

The absorbance was measured for each concentration of the primary antibody. The experiment was conducted in three sets, three times for each, and the average absorbance was calculated from the average values of the three sets.

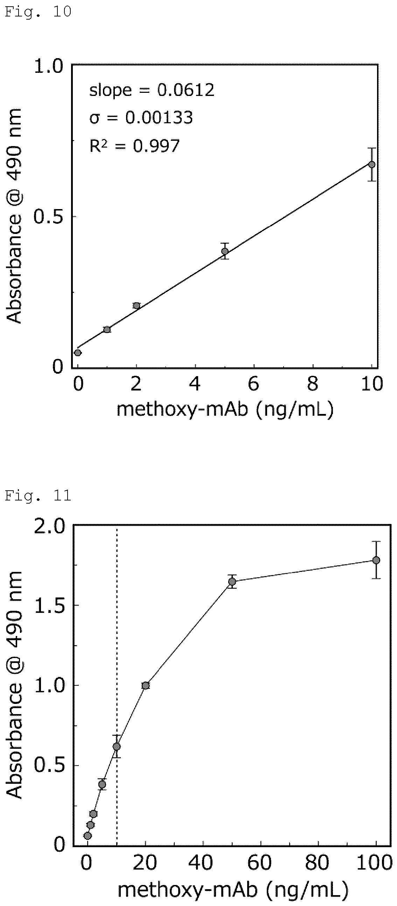

The results are as shown in Figs. 9 and 10. The horizontal axis indicates the concentration of the primary antibody (methoxy-mAb) in a reaction system in the antigen-antibody reaction of the primary antibody, whereas the vertical axis indicates the absorbance at 490 nm. Fig. 10 is a chart showing an enlarged view of the results in a low concentration range (0 to 10 ng/ml).

As shown in Figs. 9 and 10, the methoxy-terminated-PEG-selective anti-PEG antibody was found to be able to be quantitatively detected in the presence of 3% (w/v) of BSA. As shown in Fig. 10, the slope in the low concentration range was 0.0612, and standard deviation σ was 0.00133, with coefficient of determination R² being 0.997. The detection limit (=3σ/slope) was 0.065 ng/mL, indicating extremely high sensitivity.

### Test Example 8: Lower Limit for Detection in the Presence of 10% FBS

Although albumin is the most abundant protein in serum, serum also contains various other impurities, such as globulin, sugars, amino acids, urea, and metal ions, in addition to albumin. With the aim of detecting a methoxy-terminated-PEG-selective anti-PEG antibody actually present in blood, a study was performed to detect and quantify a methoxy-terminated-PEG-selective anti-PEG antibody in the presence of fetal bovine serum (FBS), used as model serum.

In Test Example 8, the cellulose dispersion for use was prepared by dispersing an OEGylated cellulose oligomer (Cell-OEG4) in PBS at a concentration of 5 mg/mL. The FBS for use was prepared by diluting 100% FBS for cell culture (manufactured by Biowest) with ultrapure water to 22 v/v% (22% FBS). The FBS contained no γ-globulin (antibody).

The primary antibody solutions for use were seven types of solutions prepared by diluting PEG-B-47 with a 6.6% (w/v) BSA/2×PBS solution to give the following primary antibody concentration: 2.2 ng/mL, 4.4 ng/mL, 11 ng/mL, 22 ng/mL, 44 ng/mL, 110 ng/mL, and 220 ng/mL. The 6.6% (w/v) BSA/2×PBS solution was prepared by dissolving 660 mg of BSA in 10 mL of 2×PBS (a solution with a double concentration of PBS, the same applies below).

The secondary antibody solution and the substrate solution used were the same as those used in Test Example 1.

### 1. Antigen-antibody Reaction of Primary Antibody

10 µL of a cellulose dispersion (Cell-OEG4, 5 mg/mL, PBS) was added to a 0.2 mL PCR tube, and 50 µL of 22% FBS was added, followed by further adding 50 µL of a primary antibody solution. After the mixture was dispersed, incubation was performed at 25°C for 1 hour to allow an antigen-antibody reaction to proceed. The antigen-antibody reaction system had a cellulose oligomer concentration of 0.45 mg/mL, a primary antibody concentration of 1 to 100 ng/mL, an FBS concentration of 10 v/v%, and a BSA concentration of 3% (w/v). For a blank, 50 µL of a 6.6% (w/v) BSA/2×PBS solution free of a primary antibody was added and incubated for 1 hour.

After incubation, centrifugation was performed (15000 rpm, 25°C, 5 minutes) to remove the supernatant, followed by redispersion in 100 µL of PBS. This cycle of centrifugation and redispersion was performed three times for washing.

### 2. Antigen-antibody Reaction of Secondary Antibody

Subsequently, centrifugation was performed (15000 rpm, 25°C, 5 minutes) to remove the supernatant, and 100 µL of a secondary antibody solution (secondary antibody content: 410 ng/mL in a 3.3% BSA/PBS solution) was added to the PCR tube and dispersed, followed by incubation at 25°C for 1 hour. Thereafter, centrifugation was performed (15000 rpm, 25°C, 5 minutes) to remove the supernatant, followed by redispersion in 100 µL of PBS. This cycle of centrifugation and redispersion was performed three times for washing.

### 3. Enzyme Reaction

Subsequently, after centrifugation was performed (15000 rpm, 25°C, 5 minutes) to remove the supernatant, 100 µL of a substrate solution (OPD: 2 mg/mL, H₂O₂: 0.11 v/v%, a citrate-phosphate buffer with a pH of 4.0) was added to the PCR tube and dispersed, followed by incubation at 25°C for 30 minutes. Thereafter, 100 µL of 3N sulfuric acid was added, and the mixture was centrifuged (15000 rpm, 25°C, 10 minutes). The supernatant was taken and diluted 5-fold with ultrapure water. 100 µL of the diluted supernatant was then added to a 96-well plate, and 100 µL of the centrifuged supernatant was added, followed by measuring the absorbance at 490 nm with a plate reader (Synergy H1, manufactured by BioTek).

The absorbance was measured for each concentration of the primary antibody. The experiment was conducted in three sets, three times for each, and the average absorbance was calculated from the average values of the three sets.

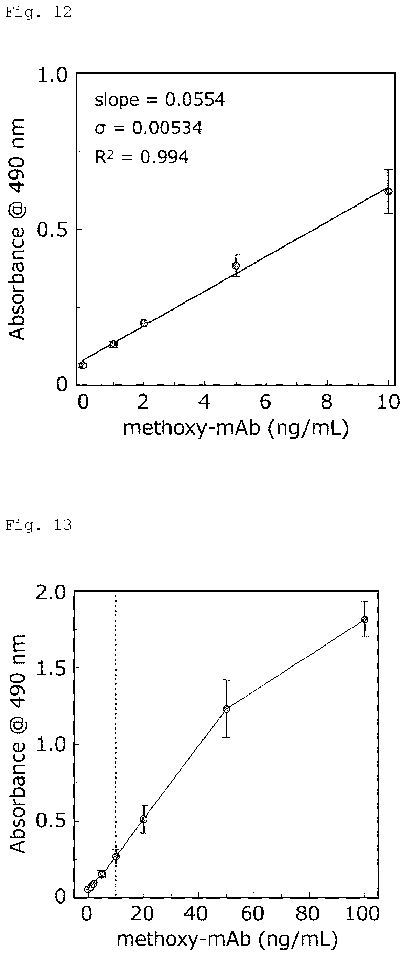

The results are as shown in Figs. 11 and 12. The horizontal axis indicates the concentration of the primary antibody (methoxy-mAb) in the reaction system in the antigen-antibody reaction of the primary antibody, whereas the vertical axis indicates the absorbance at 490 nm. Fig. 12 is a chart showing an enlarged view of the results in a low concentration range (0 to 10 ng/ml).

As shown in Figs. 11 and 12, the methoxy-terminated-PEG-selective anti-PEG antibody was found to be able to be quantitatively detected in the presence of both 10 v/v% of FBS and 3% (w/v) of BSA. As shown in Fig. 12, the slope in the low concentration range was 0.0554, and standard deviation σ was 0.00534, with coefficient of determination R² being 0.994. The detection limit (=3σ/slope) was 0.29 ng/mL, indicating extremely high sensitivity compared with about 10 ng/mL of a previously reported sensing system in the presence of serum (20-fold dilution) (Shaoyi J. et al., Anal. Chem., 2017, 89, 16, 8217-8222).

### Test Example 9: Lower Limit for Detection in the Presence of 10% FBS

To confirm the effect of adding BSA, an experiment was conducted without adding BSA in the same manner as in Test Example 8.

In Test Example 9, the primary antibody solutions for use were seven types of solutions prepared by diluting PEG-B-47 with 2×PBS so as to give the following primary antibody concentrations: 2.2 ng/mL, 4.4 ng/mL, 11 ng/mL, 22 ng/mL, 44 ng/mL, 110 ng/mL, and 220 ng/mL. The cellulose dispersion, 22% FBS, secondary antibody solution, and substrate solution were the same as those used in Test Example 8.

### 1. Antigen-antibody Reaction of Primary Antibody

10 µL of a cellulose dispersion (Cell-OEG4, 5 mg/mL, PBS) was added to a 0.2 mL PCR tube, and 50 µL of 22% FBS was added, followed by further adding 50 µL of a primary antibody solution. After the mixture was dispersed, incubation was performed at 25°C for 1 hour to allow an antigen-antibody reaction to proceed. The antigen-antibody reaction system had a cellulose oligomer concentration of 0.45 mg/mL, a primary antibody concentration of 1 to 100 ng/mL, and an FBS concentration of 10 v/v%. For a blank, 50 µL of 2×PBS free of a primary antibody was added and incubated for 1 hour.

After incubation, centrifugation was performed (15000 rpm, 25°C, 5 minutes) to remove the supernatant, followed by redispersion in 100 µL of PBS. This cycle of centrifugation and redispersion was performed three times for washing.

### 2. Antigen-antibody Reaction of Secondary Antibody

Subsequently, centrifugation was performed (15000 rpm, 25°C, 5 minutes) to remove the supernatant, and 100 µL of a secondary antibody solution (secondary antibody content: 410 ng/mL in a 3.3% BSA/PBS solution) was added to the PCR tube and dispersed, followed by incubation at 25°C for 1 hour. Thereafter, centrifugation was performed (15000 rpm, 25°C, 5 minutes) to remove the supernatant, followed by redispersion in 100 µL of PBS. This cycle of centrifugation and redispersion was performed three times for washing.

### 3. Enzyme Reaction

Subsequently, after centrifugation was performed (15000 rpm, 25°C, 5 minutes) to remove the supernatant, 100 µL of a substrate solution (OPD: 2 mg/mL, H₂O₂: 0.11 v/v%, a citrate-phosphate buffer with a pH of 4.0) was added to the PCR tube and dispersed, followed by incubation at 25°C for 30 minutes. Thereafter, 100 µL of 3N sulfuric acid was added, and the mixture was centrifuged (15000 rpm, 25°C, 10 minutes). After the supernatant was taken and diluted 5-fold with ultrapure water, 100 µL of the diluted supernatant was then added to a 96-well plate, followed by measuring the absorbance at 490 nm with a plate reader (Synergy H1, manufactured by BioTek).

The absorbance was measured for each concentration of the primary antibody. The experiment was conducted in three sets, three times for each, and the average absorbance was calculated from the average values of the three sets.

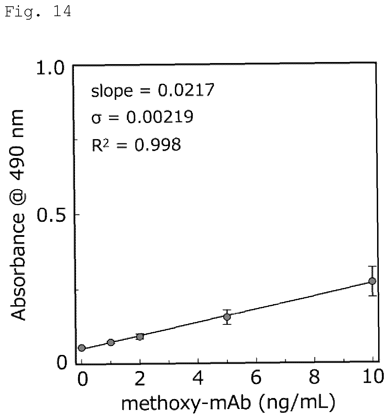

The results are as shown in Figs. 13 and 14. The horizontal axis indicates the concentration of the primary antibody (methoxy-mAb) in the reaction system of the antigen-antibody reaction of the primary antibody, whereas the vertical axis indicates the absorbance at 490 nm. Fig. 14 is a chart showing an enlarged view of the results in a low concentration range (0 to 10 ng/ml).

As shown in Figs. 13 and 14, the methoxy-terminated-PEG-selective anti-PEG antibody was found to be able to be quantitatively detected in the presence of 10 v/v% of FBS even when BSA was not added. As shown in Fig. 14, the slope in the low concentration range was 0.0217, and standard deviation σ was 0.00219, with coefficient of determination R² being 0.9979. The lower limit for detection (=3σ/slope) was 0.30 ng/mL, which was substantially equivalent to that in the presence of 3% (w/v) of BSA. Thus, the effect of adding BSA was not observed in the presence of FBS. However, the detection limit indicated extremely high sensitivity compared with 10 ng/mL of the previously reported sensing system in the presence of serum (20-fold dilution).

The numeric ranges stated in the present specification can be freely combined with their upper and lower limits, and all such combinations are regarded as disclosed in the specification as preferred numeric ranges. The expression of a numeric range "X to Y" means a range of X or more and Y or less.

Although several embodiments of the present invention are described above, these embodiments are presented as examples with no intention to limit the scope of the invention. These embodiments can be implemented in various other forms and may have various omissions, replacements, and modifications without departing from the spirit of the invention. These embodiments as well as omissions, replacements, and modifications in these embodiments fall within the scope of the invention and its spirit, and also fall within the scope of equivalents of the claimed invention.

## Claims

1. An anti-PEG-antibody-binding material capable of binding to a methoxy-terminated-PEG-selective anti-PEG antibody, comprising a cellulose oligomer represented by the following 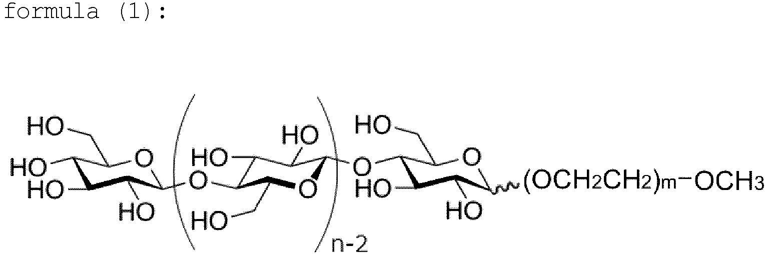 wherein n represents an average degree of polymerization, which is a number of 6 to 16, and m represents an integer of 1 to 16.

2. A biosensor comprising the anti-PEG-antibody-binding material according to claim 1.

3. An adsorbent comprising the anti-PEG-antibody-binding material according to claim 1.

4. A method for detecting an anti-PEG antibody, comprising
incubating a cellulose oligomer with a test sample suspected of containing the anti-PEG antibody under the conditions that the cellulose oligomer is bindable to a methoxy-terminated-PEG-selective anti-PEG antibody,
the cellulose oligomer being represented by the following formula (1): 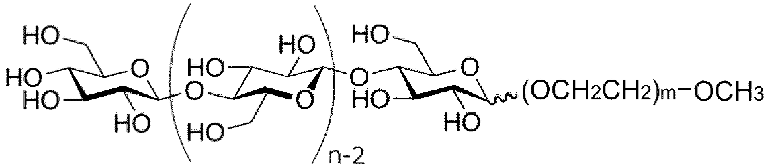
wherein n represents an average degree of polymerization, which is a number of 6 to 16, and m represents an integer of 1 to 16;
incubating the cellulose oligomer that has been incubated with the test sample with a reagent containing a secondary antibody under the conditions that the secondary antibody is bindable to the anti-PEG antibody; and
detecting the anti-PEG antibody contained in the test sample with the secondary antibody as a probe by using the cellulose oligomer that has been incubated with the reagent.
